(19) **Europäisches Patentamt**
**European Patent Office**
**Office européen des brevets**

(11) **EP 2 172 232 B1**

(12) **EUROPEAN PATENT SPECIFICATION**

(45) Date of publication and mention
of the grant of the patent:
**27.11.2019 Bulletin 2019/48**

(51) Int Cl.:
*A61L 29/14* (2006.01)        *C10M 159/12* (2006.01)
*A61L 29/08* (2006.01)

(21) Application number: **08777362.8**

(22) Date of filing: **19.06.2008**

(86) International application number:
**PCT/JP2008/061201**

(87) International publication number:
**WO 2008/156132 (24.12.2008 Gazette 2008/52)**

(54) **SURFACE COATING COMPOSITION HAVING EXCELLENT DURABILITY WHICH IS LUBRICATIVE IN WET STATE, COATING LIQUID, SURFACE COATING FILM, SURFACE COATING METHOD, AND MEDICAL DEVICE HAVING THE SURFACE COATING FILM**

ZUSAMMENSETZUNG ZUR OBERFLÄCHENBESCHICHTUNG MIT HERVORRAGENDER HALTBARKEIT, DIE IM NASSEN ZUSTAND GLEITFÄHIG IST, BESCHICHTUNGSFLÜSSIGKEIT, OBERFLÄCHENBESCHICHTUNGSFILM, OBERFLÄCHENBESCHICHTUNGSVERFAHREN UND MEDIZINPRODUKT MIT DEM OBERFLÄCHENBESCHICHTUNGSFILM

COMPOSITION DE REVÊTEMENT DE SURFACE PRÉSENTANT UNE EXCELLENTE DURABILITÉ ET QUI EST LUBRIFIANTE À L'ÉTAT HUMIDE, LIQUIDE DE REVÊTEMENT, FILM DE REVÊTEMENT DE SURFACE, PROCÉDÉ DE REVÊTEMENT DE SURFACE ET DISPOSITIF MÉDICAL PORTANT LE FILM DE REVÊTEMENT DE SURFACE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **21.06.2007 JP 2007163833**

(43) Date of publication of application:
**07.04.2010 Bulletin 2010/14**

(73) Proprietor: **Kaneka Corporation**
**Osaka 530-8288 (JP)**

(72) Inventors:
• **MATSUMURA, Yoichi**
**Settsu-shi**
**Osaka 566-0072 (JP)**

• **YAMAGUCHI, Youichi**
**Settsu-shi**
**Osaka 566-0072 (JP)**

(74) Representative: **Gille Hrabal**
**Brucknerstrasse 20**
**40593 Düsseldorf (DE)**

(56) References cited:
**WO-A1-94/23771        WO-A1-2006/095766**
**JP-A- 04 227 671      JP-A- 11 319 071**
**JP-A- 11 506 375      JP-A- 2004 280 086**
**US-A- 4 876 126       US-A- 5 306 798**
**US-A- 6 120 904**

**Description**

TECHNICAL FIELD

The present invention relates to a surface coating composition having excellent durability which is lubricative in wet state, a coating liquid, a surface coating, a surface coating method, and a medical device having the surface coating.

BACKGROUND ART

Hitherto, a lubricative surface coating in wet state is provided to medical devices containing a region that becomes in contact with the body, for reduction of the pain and the damage to the body caused by the friction during contact. However, conventional surface coatings were generally less compatible with the materials constituting the medical devices and had shortcomings such as leaching of the coating into a body and exfoliation of the coating.

For example, a method that involved treating the surface of a material with ozone to form a functional group on the surface of a base material and reacting a hydrophilic polymer with the base material by graft polymerization, as disclosed in Patent Document 1, and a method by plasma treatment, as disclosed in Patent Document 2, are known as the means to overcome the shortcomings. However, such surface coatings are unfavorably lower in durability and had adverse reactions such as deterioration in mechanical properties of the base material.

As disclosed in Patent Document 3, a method of preparing a second polymer compound by polymerization of monomers in the presence of a hydrophilic polymer compound and thus forming an interpenetrating network structure of two kinds of polymer compounds on the base material surface was proposed, but it also had problems such as deterioration of the base material and leaching of residual monomers and oligomers.

Alternatively, it is possible by a method of blending a hydrophilic polymer with a urethane resin adhesive to the base material, as disclosed in Patent Document 4, or by a method of using a urethane resin having a hydrophilic skeleton, as disclosed in Patent Document 5, to prevent the deterioration in physical properties of the base material, but there was still problems such as insufficient adhesiveness to the base material and insufficient lubricity in wet state.

It is possible to overcome the problems above by a method of using a hydrophilic polyurethane containing block isocyanates with the terminal isocyanate groups protected with a protecting group, as disclosed in Patent Document 6, but the method had additional problems to overcome such as deterioration in safety because of the need for a catalyst, for example of a heavy metal such as tin or an amine, for generation of urethane bonds on the base material and adverse effects on the mechanical properties of the base material and the profile of the medical device because of high temperature needed in processing.

A method of forming a hydrophilic urethane coating in reaction of a hydrophilic alkyleneoxide-containing urethane compound on the base material surface is known, as described in Patent Document 7, but the reaction rate is not sufficiently high, causing a problem that adhesion between the coating surfaces and also between the coating surface and the package is induced by the unreacted isocyanate. Long-term heating or curing is needed for prevention of it and thus the method is unfavorable from the viewpoint of the influence on the properties of the base material and productivity. Use of a reaction catalyst for prevention of it was also disadvantageous that it has adverse effects on biological safety.

Patent Document 1: JP-A No. 5-76590
Patent Document 2: JP-A No. 5-168695
Patent Document 3: JP-A No. 8-33704
Patent Document 4: JP-A No. 11-22613
Patent Document 5: JP-A No. 11-506375
Patent Document 6: JP-A No. 10-231347
Patent Document 7: JP-A No. 4-227671

US 5,306,798 A discloses a transparent, autoclavable, non-cytotoxic, essentially compact polyurethane embedding composition, which is prepared by reacting

a) modified diphenylmethane diisocyanate with
b) compounds with at least two reactive hydrogens in the presence of absence of
c) catalysts.

WO 94/23771 A discloses a method for improving medical or laboratory devices to increase the biocompatibility and resistance to protein binding.

US 4,876,126 discloses a medical instrument comprising a substrate inherently having a reactive functional group on a surface thereof; and a water-soluble polymer selected from the group consisting of a cellulosic polymer, a maleic anhydride polymer, and a water-soluble nylon, or a derivative thereof.

US 6,120,904 A discloses a medical device comprising an organic polymer substrate material having a coating thereon. The coating comprises an interpenetrating network of two different hydrogel polymers.

JP 04/227671 A discloses polyethylene oxide having an average molecular weight of 300000 as a corresponding compound of (c).

JP 2004/280086 A discloses surface coatings having high durability and showing good wet-lubricity; methods for surface coating; and medical devices having the same.

WO 2006/095766 A discloses a surface coating comprising

a) an urethane polymer layer which includes (a) 40 to 80 % by weight of at least one component selected from an aromatic diisocyanate, an aliphatic diisocyanate and an alicyclic diisocyanate and (b) 20 to 60 % by weight of a polyol having at least trifunctionality, and

b) a hydrophilic polymer layer which is provided as an outer layer for the urethane polymer layer.

Disclosure of the Invention

Problems to be Solved by the invention

An object of the present invention is to provide a highly lubricative surface coating in wet state and superior in durability, in particular a surface coating that can be formed on a base material having no particular functional group on the surface and is superior in biological safety.

Means to solve the problems

After intensive studies under the circumstances above, the inventors have found that the problems above are overcome by the following configurations.

Specifically, the present invention relates to a surface-coating composition containing the following composition (A):

(A) a composition containing

(a) at least one of an aromatic diisocyanate, an aliphatic diisocyanate and an alicyclic diisocyanate in an amount of 1 to 35 wt % in the surface-coating composition,

(b) a trifunctional or higher functional polyol in an amount of 1 to 35 wt % in the surface-coating composition, selected from a group consisting of branched polymeric polyols of polyester-based polyols; poly(oxypropylene ether)polyol, poly (oxyethylene-propylene ether)polyol and polytetramethylene glycol; acrylic polyols, castor oil, glycerol, trimethylolpropane, trimethylolethane, 1,2,6,-hexanetriol, pentaerythritol, sorbitol and mannitol, and

(c) a polyethylene glycol in an amount of 30 to 98 wt % in the surface-coating composition, having a weight-average molecular weight of 4000 to 40000;

wherein the above-mentioned compounds (a), (b) and (c) are present with respect to 100 wt % of the total amount of components (a), (b) and (c) in the surface coating composition.

The present invention also relates to a coating liquid prepared by dissolving or suspending the above-mentioned surface coating composition in a volatile organic solvent.

The present invention also relates to a surface coating being prepared by coating the above-mentioned liquid on the surface of an object base material and to a medical device having the surface coating.

The present invention also relates to a method of forming a surface coating and also to a method of producing a medical device, comprising coating and drying the above-mentioned coating liquid on the surface of an object base material. Finally, the present invention relates to a medical device, which is produced by the afore-mentioned method of forming a surface coating or the method of producing a medical device.

Effects of the Invention

Advantageously, according to the present invention, a highly lubricative surface coating in wet state and superior in durability can be prepared by a simple and convenient method.

BEST MODE OF CARRYING OUT THE INVENTION

Hereinafter, the present invention will be described in detail.

The present invention relates to a surface-coating composition comprising the following composition (A):

(A) a composition containing

(a) at least one of an aromatic diisocyanate, an aliphatic diisocyanate and an alicyclic diisocyanate in an amount of 1 to 35 wt % in the surface-coating composition,

(b) a trifunctional or higher functional polyol in an amount of 1 to 35 wt % in the surface-coating composition, selected from a group consisting of branched polymeric polyols of polyester-based polyols; poly(oxypropylene ether)polyol, poly (oxyethylene-propylene ether)polyol and polytetramethylene glycol; acrylic polyols, castor oil, glycerol, trimethylolpropane, trimethylolethane, 1,2,6,-hexanetriol, pentaerythritol, sorbitol and mannitol, and

(c) a polyethylene glycol in an amount of 30 to 98 wt % in the surface-coating composition, having a weight-average molecular weight of 4000 to 40000;

wherein the above-mentioned compounds (a), (b) and (c) are present with respect to 100 wt % of the total amount of components (a), (b) and (c) in the surface coating composition.

It also relates to a coating liquid containing the surface-coating composition or the reaction products thereof, a surface coating obtained by applying it on the surface of an object base material, a medical device having the surface coating, as well as methods of forming and producing these.

In the present invention, the thickness of the coating is not particularly limited, but preferably 0.5 to 30 [μm], particularly preferably 0.5 to 10 [μm], and more preferably 0.5 to 5 [μm], when it is used in a medical device. A coating thickness of smaller than the range above unfavorably leads to deterioration in durability and also in lubricity, when the coating is placed under friction. On the other hand, excessively large thickness unfavorably leads to increase in the amount of the leachate in fluids such as blood.

The surface-coating composition according to the present invention is configured to contain at least one compound selected from an aromatic diisocyanate, an aliphatic diisocyanate and an alicyclic diisocyanate in an amount of 1 to 35 wt % in the surface-coating composition (hereinafter, referred to as diisocyanate (a) or simply as compound (a)). These compounds normally have two isocyanate groups as functional groups in the molecule.

Examples of the aromatic diisocyanates include 2,4-tolylene diisocyanate, 2,6-tolylene diisocyanate, 4,4'-diphenylmethane diisocyanate, p-phenylene diisocyanate, 3,3'-dimethylphenyl-4,4'-diisocyanate, m-xylylene diisocyanate, dianisidine diisocyanate, m-xylene diisocyanate, tetramethylxylene diisocyanate, 1,5-naphthalene diisocyanate and the like.

Examples of the aliphatic diisocyanates include trans-vinylene diisocyanate, 2,2,4-trimethylhexamethylene diisocyanate, lysine diisocyanate, 1,6-hexamethylene diisocyanate and the like.

Examples of the alicyclic diisocyanates include trans-1,4-cyclohexane diisocyanate, cis-1,4-cyclohexane diisocyanate, 4,4'-dicyclohexylmethane diisocyanate, isophorone diisocyanate and the like. These isocyanates may be used alone or in combination of two or more.

The content of the at least one of an aromatic diisocyanate, an aliphatic diisocyanate and an alicyclic diisocyanate is 1 to 35 wt % in the surface-coating composition, , preferably of 2 wt % or more and 30 wt % or less, and more preferably of 2 wt % or more and 25 wt % or less, with respect to 100 wt% of the total amount with the compounds (b) and (c) described below. A diisocyanate (a) content of less than the range above unfavorably leads to deterioration in the adhesive strength to the base material and also in the durability of the coating under friction. On the other hand, a diisocyanate (a) content of larger than the range above unfavorably leads to increase in fragility of the coating agent.

In addition, the surface-coating composition according to the present invention is configured to contain (b) a trifunctional or higher functional polyol in an amount of 1 to 35 wt % in the surface-coating composition, selected from a group consisting of branched polymeric polyols of polyester-based polyols; poly(oxypropylene ether)polyol, poly (oxyethylene-propylene ether)polyol and polytetramethylene glycol; acrylic polyols, castor oil, glycerol, trimethylolpropane, trimethylolethane, 1,2,6,-hexanetriol, pentaerythritol, sorbitol and mannitol (hereinafter, referred to simply as compound (b)). Compound (b) is a practically trifunctional or higher functional polyol having more than two hydroxy groups. The present invention demands a trifunctional or higher functional polyol especially as the essential component, and the presence of it is effective for improvement in durability of the surface coating and especially for prevention of increase in friction coefficient under friction.

It was first considered that the structure (b) is not involved in expression of durability in wet lubricity, because a polyethylene glycol in an amount of 30 to 98 wt % in the surface-coating composition, having a weight-average molecular weight of 4000 to 40000 (hereinafter, referred to simply as component (c)), is mainly responsible for expression of wet lubricity, but it had significant contribution to the durability in practice. The inventors consider that it is because presence particularly of a trifunctional or higher functional polyol leads to crosslinking of the polymer chain, resulting in reduction in the amount of the polymer chains that are not involved in lubricity and durability because of a significant amount of leaching when swollen in the polymer chains containing the component (c) that are produced by conventional methods and in increase in physical entanglement of the unreacted component (c) to the coating by conversion into the three-dimensional structure.

In addition, it was difficult to obtain a coating durable enough to satisfy the requirement demanded for example for medical devices, by coating a conventional lubricative surface coating composition in wet state after polymerization on the surface of an object base material surface, and thus, the polymerization of the coating should be carried out on the surface of the object base material. However, because a processing such as heating for the polymer reaction leads to deterioration in mechanical properties of the base material and deformation and decrease in controlled orientation of the base material, the scope of its application was limited. In contrast, the surface-coating composition according to the present invention satisfies the requirement in durability demanded for medical devices, even if the reaction product is used for coating.

Examples of the compound (b) are a trifunctional or higher functional polyol in an amount of 1 to 35 wt % in the surface-coating composition, selected from a group consisting of branched polymeric polyols of polyester-based polyols; poly(oxypropylene ether)polyol, poly (oxyethylene-propylene ether)polyol and polytetramethylene glycol; acrylic polyols, castor oil, glycerol, trimethylolpropane, trimethylolethane, 1,2,6,-hexanetriol, pentaerythritol, sorbitol and mannitol. The molecular weight of the branched derivative of polymer polyol is preferably in the range of 200 or more and 40000 or less, more preferably of 200 or more and 5000 or less, and still more preferably of 200 or more and 3000 or less. A molecular weight of larger than the range above unfavorably leads to deterioration in the adhesive strength of the coating formed, while a molecular weight of smaller than the range above to deterioration in flexibility of the coating formed. These polyols may be used alone or as a mixture of two or more.

The content of the compound (b) in the surface-coating composition according to the present invention is in the range of 1 wt % or more and 35 wt % or less, preferably of 2 wt % or more and 30 wt % or less, and more preferably 2 wt % or more and 25 wt % or less, with respect to 100 wt% of the total amount with the compound (a) and the compound (c). The compound (b) content of smaller than the range above unfavorably leads to deterioration in adhesive strength to the base material and also in durability of the coating under friction. The compound (b) content of more than the range above unfavorably leads to greasy feeling and also tacking of the coating.

The compound (a) and the compound (b) may be added, after part of them are brought into reaction with each other, for example, to give a urethane prepolymer. In this case, the contents of the compounds (a), (b) and (c) are calculated, based on the amount of respective components before reaction. The amounts of the compound (b) and (c) used in forming the prepolymer in reaction with the component (a) are such that the coating composition in the scope of the present invention is obtained. The ratio of (a)/(b) is preferably 3/1 to 1/3 by weight. A component (b) ratio of more than the range above leads to hardening of the prepolymer itself and reduction of the reaction between the isocyanate groups of the component (a) and the component (c), while a ratio of less than the region unfavorably leads to deterioration in handling efficiency of the reactive prepolymer. The ratio of (a)/(c) favorably used is in the range of 3/1 to 1/5 by weight. A component (c) ratio of more than the range above unfavorably leads to decrease in the amount of the component (a) isocyanate groups in reaction with the component (b) and thus, decrease of three-dimensional crosslinking, while a ratio of less than the range above is also unfavorable, because it leads to deterioration in handling efficiency of the reactive prepolymer. When the components (a), (b) and (c) are added simultaneously in forming a prepolymer, each of components is used favorably in an amount in the range above.

In addition, the surface-coating composition according to the present invention is configured to contain a component (c).

Component (c) is a polyethylene glycol.

The weight-average molecular weight of the polyethylene glycol is 4000 to 40000. The weight-average molecular weight of the compound (c) for use in the present invention is particularly preferably 40000 or less. When the physical entanglement of the component (c) to the polymer from components (a) and (b) is considered, the weight-average molecular weight of the compound (c) would be preferably more than 50000. However after studies by the inventors, it was found surprisingly that it was possible to improve drastically the durability of the surface coating, especially the resistance to increase in friction coefficient under friction, especially by controlling the weight-average molecular weight of the component (c) to 40000 or less. According to the view of the inventors, it is because the adhesion of the component (c) to the surface coating is caused by physical entanglement of the polymer of the components (a) and (b) and also by the chemical binding force and it is possible to improve the durability more efficiently by raising the rate of the chemical binding force than by increasing the physical entanglement (decrease in molecular weight would lead to increase in migratory efficiency of the component (c) and the number of the terminal reactive groups and thus in reactivity). On the other hand, decrease in molecular weight of the compound (c) to less than the range above leads to deterioration in lubricity and also durability of the surface coating, and thus, use of such a compound is unfavorable.

When the compound (c) is a multifunctional polyethylene glycol derivative and the number of the terminal hydroxy groups is designated as n, the weight-average molecular weight thereof is preferably in the range satisfying the following Formula:

$$25000 \times n > \text{Weight-average molecular weight} > 1000 \times n \quad \text{(Formula)}$$

A weight-average molecular weight of lower than the range above unfavorably leads to deterioration in wet lubricity, while that larger than the range above unfavorably leads to roughening and whitening of the coating surface.

The content of the compound (c) in the surface-coating composition according to the present invention is in the range of 30 wt % or more and 98 wt % or less, preferably of 40 wt % or more and 98 wt % or less, more preferably of 50 wt % or more and 95 wt % or less, and more preferably of 60 wt % or more and 90 wt % or less, wherein the above-mentioned compounds (a), (b) and (c) are present with respect to 100 wt % of the total amount of components (a), (b) and (c) in the surface coating composition. A content of the polyethylene glycol component of less than the range above unfavorably leads to deterioration in lubricity, while a content of more than the range above unfavorably leads to deterioration in durability and lubricity of the coating under friction.

It is particularly preferable from the point of availability of the raw materials to add a polyethylene glycol (c) to the reaction product of an at least one of an aromatic diisocyanate, an aliphatic diisocyanate and an alicyclic diisocyanate in an amount of 1 to 35 wt % in the surface-coating composition (a) and a trifunctional or higher functional polyol in an amount of 1 to 35 wt % in the surface-coating composition, selected from a group consisting of branched polymeric polyols of polyester-based polyols; poly(oxypropylene ether)polyol, poly (oxyethylene-propylene ether)polyol and polytetramethylene glycol; acrylic polyols, castor oil, glycerol, trimethylolpropane, trimethylolethane, 1,2,6,-hexanetriol, pentaerythritol, sorbitol and mannitol (b) and allow them to react with each other. Compounds (a) containing a small amount of a compound (b) are commercially available and can be used favorably.

On the other hand, if the reaction above is insufficient, the isocyanate group remains in the surface coating formed, and there is a concern about adhesion between the coated surfaces or between the coated material and its package or protective material. Accordingly, the method of reacting the compound (a), the compound (b), and the compound (c) with each other is not particularly limited, if a desired reaction product can be obtained, but a reaction in solution system is used favorably for prevention of insufficient reaction and from the viewpoints of reaction and homogeneous dispersion of the composition. The solvent used then is preferably an organic solvent having no active hydrogen reactive with the isocyanate group. Examples of such solvents include acetonitrile, THF, acetone, halogenated hydrocarbons such as dichloromethane and chloroform, and the like.

In addition, a compound having an active hydrogen atom, more preferably a low-molecular weight compound having an active hydrogen atom is added after practical termination of the reaction of the compounds (a), (b) and (c) in the surface composition, for more effective inactivation of the residual isocyanate groups in the surface-coating composition. Here, the state after the practical termination of reaction means a state in which most of the theoretically possible reactive functional groups according to the numbers of the functional groups respectively of the compounds (a), (b) and (c) added to the surface-coating composition are consumed in reaction. The state also includes the case when excessive amounts of functional groups of more than that needed for obtaining desired properties are introduced into the system and a compound having an active hydrogen atom is added during the reaction, but the method is not a favorable embodiment, because it prohibits easy control of the reaction. Favorable examples of the active hydrogen compounds include alcohols, amines, carboxylic acids and the like, and alcohols are used favorably, from the viewpoint of stability. Examples of the alcohols include alkyl alcohols such as methanol, ethanol and isopropanol; polyols such as glycerol and pentaerythritol; and the like.

Use of water as the active hydrogen compound, which often results in generation of insoluble urea and bubbles by generation of urea bonds, is unfavorable from the viewpoints of deterioration in stability and lubricity of the solution.

The method of determining the amount of the residual isocyanate does not exert any influence on the present invention, and thus, any existing means may be used. An example thereof is the titration specified by ASTM D1638-74.

The method of coating the wet lubricating surface is not particularly limited, if a surface coating within the scope of the present invention is obtained, and examples thereof include a method of applying the solution or suspension of the coating materials (surface-coating composition or the reaction product) on the surface of a material and making it absorbed on the material surface, a method of dissolving or suspending the components in a volatile organic solvent such as tetrahydrofuran, coating the solution or suspension on the surface of a material by means such as dipping or spraying, evaporating the volatile organic solvent as it is left still, and thus, forming a thin coating on the material surface, and the like. Methods of dissolving the components in a volatile nonaqueous solution, particularly in an organic solvent, are used favorably, from the points of uniformity of coating and easiness of solvent removal.

On the other hand, the solvent for the coating liquid used in forming the surface coating is not particularly limited, but, for prevention of the degradation of urethane over time, a nonaqueous solvent having no active hydrogen but having a suitable affinity to the surface of the coating base material and a solubility parameter $\delta$ of 8 to 13 [(cal/cm)$^{1/2}$] is preferable. Preferable are nonaqueous solvents having a solubility parameter $\delta$ of 9 to 12 such as tetrahydrofuran and acetone.

The surface coating formed according to the present invention may contain, in addition to the polymer obtained in reaction of the surface-coating composition and the solvent, various ingredients commonly used in the field such as pharmaceutical components, blood anticoagulants, pharmaceutical components, disintegrants, pharmaceutical component absorption accelerators, plasticizers, stabilizers, radiation ray absorbents and as needed polymer compounds other than those above.

In addition, a hydrophobic oils such as silicone oil or functionalized silicone oil may be applied further on the coating surface as blocking inhibitor.

The medical device according to the present invention is a device used in contact for example with biological component such as body tissue or body fluid. Typical examples of the medical devices include, but are not limited to, blood bag, urine-collecting bag, blood transfusion set, surgical suture, drain tube, various catheters, blood access, blood circuit, artificial vessel, dialyzer, cardio-pulmonary pump, artificial valves, blood plasma-exchange membrane, various adsorbents, CAPD, IABP, pacemaker, artificial joint, synthetic caput, dental materials, intraocular lens, soft contact lens, various shunts and the like.

The raw material for the medical device is not particularly limited, and polyalkylenes, polyamides, various elastomers such as polyamide elastomers, polyesters, polycarbonates, polyurethanes, polyvinyl chlorides, silicones and the like are used favorably.

All of the surface of the medical device may show lubricity in wet state, or alternatively, only part of the surface, for example in contact with a biological component such as body tissue or body fluid, may show lubricity in wet state. It is also possible as needed to make two or more lubricative regions different in lubricity, by changing the coating amount by means of modifying the concentration of the coating liquid and the number of applications.

EXAMPLES

Hereinafter, the invention will be described specifically with reference to Examples, but it should be understood that the present invention is not restricted at all by these Examples.

Abbreviations used in the following description are as follows:

4,4'-MDI: 4,4'-diphenylmethane diisocyanate
2,4-TDI: 2,4-tolylene diisocyanate
1,6-HDI: 1,6-hexamethylene diisocyanate
Hydrogenated MDI: methylene bis(4,1-cyclohexylene) diisocyanate (4,4'-methylene bis(cyclohexylisocyanate))
PPG700: polypropylene glycol (triol derivative), weight-average molecular weight: 700
PEG600: polyethylene glycol (diol derivative), weight-average molecular weight: 800
PEG20000: polyethylene glycol, weight-average molecular weight: 20000
PEG4000: polyethylene glycol, weight-average molecular weight: 4000
PEG500000: polyethylene glycol, weight-average molecular weight: 500000
MEPEG4000: monomethoxypolyethylene glycol, weight-average molecular weight: 4000
PEG400: polyethylene glycol, weight-average molecular weight: 400
PVP90: polyvinylpyrrolidone
Pebax: polyamide elastomer resin, manufactured by Elf Atochem

The part and the % used in Examples and Comparative Examples are wt part and wt % respectively, unless specified otherwise. The tests and evaluations in Examples and Comparative Examples were made under the following conditions and by the following methods.

[Quantitative determination of residual isocyanate groups]

The amount of residual isocyanate groups was determined according to the back titration specified by ASTM D1638-74.

[Measurement of friction coefficient and durability]

A hard vinyl chloride friction block having a diameter of 10 [mm] as the friction block was slid on a test piece under a load of 100 [gf] at a speed of 100[mm/sec] and a stroke of 30[mm] in a friction tester manufactured by HEIDON, and the friction coefficient after 10 reciprocations was determined for evaluation of the lubricity. The friction coefficient after 100 reciprocations was also determined and compared with that after 10 reciprocations, for evaluation of the durability according to the following criteria:

○; No change in friction coefficient observed
△; Increase in friction coefficient observed
×; Increase in friction coefficient to the value before coating or more

[Appearance]

The appearance of the coating was evaluated by visual observation.

○; No uneven coating or whitening observed
△; Some uneven coating or whitening observed.
×; Significant uneven coating or whitening observed

[SEM observation]

A coated tube was cut crosswise, and the cross-sectional surface was observed under a scanning electron microscope for evaluation of the thickness of the coating.

[Adhesion]

A bundle of coated tubes was placed under a weight of 100 g and stored at 55°C and 100% RH for 48 hours. The degree of adhesion between the tubes was evaluated.

○; No adhesion observed
△; Some adhesion observed
×; Significant adhesion observed.

(Example 1)

(1) Preparation of solution A

The following raw materials were placed in a 1L round-bottomed flask.

4,4'-Diphenylmethane diisocyanate 60 [g]
Dry THF 567 [g]
Polypropylene glycol 700 40 [g]

The mixture was stirred under nitrogen stream until homogeneity, to give a (solution A).

(2) Preparation of coating liquid

Then, 25 [g] of solution A was added to a solution (solution B) of 50[g] of polyethylene glycol having a weight-average molecular weight of 20000 in 950 [g] of dry THF, and the mixture was stirred under nitrogen stream, as heated to 40°C for 3 hours, to give a coating liquid. The amount of the residual isocyanate groups in the coating liquid was found to be 0%.

(3) Formation of coating (surface coating)

A Pebax tube having an external diameter of 1 [mm] and a length of 200 [mm] was immersed into the coating liquid obtained, withdrawn from it in the direction in parallel with the tube length direction at a constant speed of 10 [mm/sec], and dried at room temperature for 1 hour, to give a polyamide elastomer tube having a coating. The tube was cut along a plane perpendicular to the length direction, and the cross section was observed under a scanning electron microscope. The thickness of the coating determined was 1.5 [μm]. Evaluation results are shown in Table 1.

(Examples 2 and 3)

Tubes having a lubricative surface coating of Examples 2 and 3 were prepared by using the raw materials and the method shown in Table 1, in a manner similar to Example 1. Evaluation results are shown in Table 1.

(Examples 4 and 5)

A coating liquid was obtained by using the raw materials and the method shown in Table 1 in a manner similar to Example 1, except that ethanol was added after blending (reaction) of the lubricative surface coating composition and the mixture was stirred additionally at 40°C for 1 hour. A tube having a lubricative surface coating was prepared by using the coating liquid, in a manner similar to Example 1. Evaluation results are shown in Table 1.

(Example 6)

(1) Synthesis of polyurethane prepolymer (C-1)

The following substance was placed in a 200-mL reactor:
4,4'-Diphenylmethane diisocyanate 70 [g]
It was heated to 70°C as stirred under nitrogen stream, and the following substance was added dropwise over 2 hours, while the mixture was stirred additionally:
Polypropylene glycol 700 (triol derivative) 30[g]
The mixture was stirred for 2 hours at a constant temperature of 70°C under nitrogen stream, to give a polyurethane prepolymer (C-1). The blending composition was shown in Table 2.

(2) Preparation of coating liquid

To 85 [g] of THF, 12.75 [g] of polyurethane prepolymer (C-1) and 2.25 [g] of PPG700 were added and the mixture was stirred thoroughly to give a homogeneous solution. Then, 1000[g] of 15% THF solution of polyethylene glycol 20000 was added thereto, and the mixture was heated to 40°C and stirred for 6 hours to give a coating liquid. The amount of the residual isocyanate groups was found to be 0%.

(3) Formation of coating (surface coating)

A Pebax tube having an external diameter of 1 [mm] and a length of 200 [mm] was immersed into the coating liquid obtained, withdrawn from it in the direction in parallel with the tube length direction at a constant speed of 10 [mm/sec], and dried at room temperature for 1 hour, to give a polyamide elastomer tube having a coating. The tube was cut along a plane perpendicular to the length direction, and the cross section was observed under scanning electron microscope. The thickness of the coating determined was 2.0 [μm]. Evaluation results are shown in Table 1.

(Examples 7 to 13)

Tubes having a lubricative surface coating of Examples 7 to 13 were prepared by using the raw materials and the methods shown in Tables 1 and 2, in a manner similar to Example 6. Evaluation results are shown in Table 1. Examples 9, 11 and 12 are not according to the invention.

(Example 14)

To 85[g] of THF,12.75 [g] of polyurethane prepolymer (C-1) and 2.25 [g] of PPG700 were added and the mixture was stirred thoroughly to give a solution. Then, 1000 [g] of 15% THF solution of polyethylene glycol 20000 was added thereto, and the mixture was stirred until homogeneity. The amount of the residual isocyanate groups was found to be 73%. A Pebax tube having an external diameter of 1 [mm] and a length of 200 [mm] was immersed into the solution, withdrawn from it in the direction in parallel with the tube length direction at a constant speed of 10 [mm/sec], and dried at 75°C for 1 hour, to give a polyamide elastomer tube having a coating. The tube was shrunken and bent.

(Comparative Examples 1 to 4)

Tubes having a lubricating surface coating of Comparative Examples 1 to 4 were prepared by using the raw materials shown in Table 1 in a manner similar to Example 1. Evaluation results are shown in Table 1.

[Table 1]

| | | | Example 1 | Example 2 | Example 3 | Example 4 | Example 5 | Example 6 | Example 7 | Example 8 | Example 9 | Example 10 | Example 11 | Example 12 | Example 13 | Example 14 | Comparative Example 1 | Comparative Example 2 | Comparative Example 3 | Comparative Example 4 |
|---|---|---|---|---|---|---|---|---|---|---|---|---|---|---|---|---|---|---|---|---|
| Solution A | Isocyanate (a) | 4,4'-MDI | 60 | 50 | | | | | | | | | 50 | 50 | 50 | | 50 | 50 | 50 | 50 |
| | | 2,4-TDI | | | 45 | | | | | | | | | | | | | | | |
| | | 1,6-HDI | | | | 52 | | | | | | | | | | | | | | |
| | | Hydrogenated MDI | | | | | 55 | | | | | | | | | | | | | |
| | | Prepolymer (C-1) | | | | | | 85 | | | | | | | | 85 | | | | |
| | | Prepolymer (C-2) | | | | | | | 80 | 80 | | | | | | | | | | |
| | | Prepolymer (C-3) | | | | | | | | | 80 | | | | | | | | | |
| | | Prepolymer (C-4) | | | | | | | | | | 80 | | | | | | | | |
| | Polyol (b) [parts] | PPG700 | 40 | 50 | 55 | | | 15 | | | | | | | | 15 | | | | |
| | | Castor oil | | | | 48 | 45 | | 20 | 20 | 20 | 20 | 50 | 50 | 50 | | 50 | | 50 | 50 |
| | | PEG600 | | | | | | | | | | | | | | | | 50 | | |
| | THF solution concentration[%] | | 15% | 15% | 15% | 15% | 15% | 15% | 15% | 10% | 15% | 5% | 15% | 15% | 15% | 15% | 15% | 15% | 15% | 15% |
| Solution B | Polyalkylene glycol (c) [parts] | PEG20000 | 100 | 100 | 100 | 100 | 100 | 100 | 100 | | | 100 | | | 100 | 100 | 100 | 100 | 100 | |
| | | PEG4000 | | | | | | | | 100 | | | | | | | | | | |
| | | MEPEG4000 | | | | | | | | | 100 | | | | | | | | | |
| | | PEG500000 | | | | | | | | | | | | 100 | | | | | | |
| | | PEG400 | | | | | | | | | | | 100 | | | | | | | |
| | | PVP90 | | | | | | | | | | | | | | | | | | 100 |
| | THF solution concentration[%] | | 5% | 5% | 5% | 5% | 5% | 15% | 5% | 5% | 15% | 20% | 5% | 5% | 5% | 15% | 5% | 5% | 10% | 5% |
| Blending ratio A:B | Solution A ratio | | 1 | 1 | 1 | 1 | 1 | 1 | 1 | 1 | 1 | 1 | 1 | 1 | 1 | 1 | 1 | 1 | 1 | 1 |
| | Solution B ratio | | 40 | 20 | 10 | 5 | 2 | 10 | 20 | 8 | 2 | 1 | 10 | 20 | 20 | 10 | 1 | 10 | 80 | 10 |
| | Active hydrogen compound (ethanol) | | - | - | - | 0.01 | 0.02 | - | - | - | 0.01 | 0.02 | - | - | - | - | 0.02 | - | - | - |
| Ratio | (a) | | 4.2% | 6.5% | 10.4% | 19.5% | 33.0% | 7.6% | 10.1% | 15.2% | 24.7% | 16.0% | 11.5% | 6.5% | 6.5% | 7.6% | 37.5% | 11.5% | 0.9% | 11.5% |
| | (b) | | 2.8% | 6.5% | 12.7% | 18.0% | 27.0% | 3.6% | 6.0% | 9.1% | 13.6% | 4.0% | 11.5% | 6.5% | 6.5% | 3.6% | 37.5% | 11.5% | 0.9% | 11.5% |
| | (c) | | 93.0% | 87.0% | 76.9% | 62.5% | 40.0% | 88.8% | 83.9% | 75.8% | 61.7% | 80.0% | 76.9% | 87.0% | 87.0% | 88.8% | 25.0% | 76.9% | 98.2% | 0.0% |
| Coating film thickness[μ] | | | 1.5 | 2.0 | 2.0 | 2.5 | 2.5 | 1.5 | 2.0 | 2.0 | 1.5 | 2.5 | 2.5 | 2.5 | 2.0 | 1.0 | 2.0 | 2.5 | 3.5 | 2.5 |
| Residual isocyanate group content | | | 0.0% | 0.0% | 0.0% | 0.0% | 0.0% | 0.0% | 0.0% | 0.0% | 0.0% | 0.0% | 0.0% | 0.0% | 0.0% | 0.0% | 73% | 12.0% | 0.0% | 0.0% |
| Appearance | | | ○ | ○ | ○ | ○ | ○ | ○ | ○ | ○ | ○ | ○ | ○ | △ | ○ | △ | ○ | ○ | ○ | ○ |
| Adhesion | | | ○ | ○ | ○ | ○ | ○ | ○ | ○ | ○ | ○ | ○ | ○ | △ | ○ | △ | × | ○ | ○ | ○ |
| Friction coefficient | | | 0.03 | 0.03 | 0.04 | 0.05 | 0.22 | 0.05 | 0.03 | 0.08 | 0.05 | 0.30 | 0.35 | 0.15 | 0.03 | 0.05 | 0.65 | 0.04 | 0.12 | 0.55 |
| Durability | | | ○ | ○ | ○ | ○ | ○ | ○ | ○ | ○ | ○ | ○ | △ | △ | ○ | ○ | ○ | × | × | × |

Examples 9, 11 and 12 are not according to the invention.

[Table 2]

| | | Pre polymer | | | |
|---|---|---|---|---|---|
| | | (C-1) | (C-2) | (C-3) | (C-4) |
| Isocyanate [parts] | 4, 4' -MDI | 70 | 65 | | |
| | 1, 6 -HDI | | | 45 | |
| | Hydrogenated MDI | | | | 70 |
| Polyol [parts] | PPG700 | 30 | | 55 | 30 |
| | Castor oil | | 35 | | |

As the results in Table 1 show distinctively, the lubricative surface coating according to the present invention represented by those of Examples 1 to 14 show superior lubricity in wet state and durability, and show the superior lubricity and durability even when formed on a base material having no active hydrogen or particular functional group on the surface.

**Claims**

1.  A surface coating composition, comprising the following composition (A):

    (A) a composition containing

    (a) at least one of an aromatic diisocyanate, an aliphatic diisocyanate and an alicyclic diisocyanate in an amount of 1 to 35 wt % in the surface-coating composition,
    (b) a trifunctional or higher functional polyol in an amount of 1 to 35 wt % in the surface-coating composition, selected from a group consisting of branched polymeric polyols of polyester-based polyols; poly(oxypropylene ether)polyol, poly (oxyethylene-propylene ether)polyol and polytetramethylene glycol; acrylic polyols, castor oil, glycerol, trimethylolpropane, trimethylolethane, 1,2,6,-hexanetriol, pentaerythritol, sorbitol and mannitol, and
    (c) a polyethylene glycol in an amount of 30 to 98 wt % in the surface-coating composition, having a weight-average molecular weight of 4000 to 40000;

    wherein the above-mentioned compounds (a), (b) and (c) are present with respect to 100 wt % of the total amount of components (a), (b) and (c) in the surface coating composition.

2.  A coating liquid prepared by dissolving or suspending the surface coating composition of Claim 1 in a volatile organic solvent.

3.  The coating liquid according to Claim 2, wherein the coating liquid is a nonaqueous solution.

4.  A surface coating, **characterized by** being prepared by coating liquid according to Claim 2 or 3 on the surface of an object base material.

5.  A medical device having the surface coating according to Claim 4.

6.  A method of forming a surface coating, comprising coating and drying the coating liquid according to Claim 2 or 3 on the surface of an object base material.

7.  The method of forming a surface coating according to claim 6, wherein a compound having an active hydrogen atom is added after practical termination of the reaction of the compounds (a), (b) and (c) in the surface-coating composition.

8.  A method of producing a medical device, comprising coating and drying the coating liquid according to Claim 2 or 3 on the surface of an object base material.

9.  A medical device, **characterized by** being produced by the method of forming a surface coating according to Claim 6 or the method of producing a medical device according to Claim 8.

**Patentansprüche**

1.  Oberflächenbeschichtungszusammensetzung mit der folgenden Zusammensetzung (A):

    (A) eine Zusammensetzung, die enthält:

    (a) zumindest ein Element aus einem aromatischen Diisocyanat, einem aliphatischen Diisocyanat und einem alizyklischen Diisocyanat in einer Menge von 1 bis 35 Gew.-% in der Oberflächenbesch ichtungszusammensetzung,

(b) einen trifunktionalen oder höher funktionalen Polyalkohol in einer Menge von 1 bis 35 Gew,-% in der Oberflächenbeschichtungszusammensetzung, ausgewählt aus einer Gruppe, die besteht aus: verzweigten polymeren Polyolen Polyesterbasieten Polyolen; Poly(Oxypropylen-Ether)Polyol, Poly(Oxyethylen-Propylen-Ether)Polyol und Polytetramethylen-Glykol; akrylischen Polyolen, Rizinusöl, Glyzerin, Trimethylolpropan, Trimethylolethan, 1,2,6,-hexanetriol, Pentaerythritol, Sorbitol und Mannitol, und
(c) einen Polyethylen-Glykol in einer Mange von 30 bis 98 Gew.-% in der Oberflächenbeschichtungszusammensetzung, mit einem durchschnittlichen Molekulargewicht von 4000 bis 40000;

wobei die oben genannten Verbindungen (a), (b) und (c) bezogen auf 100 Gew.-% der Gesamtmenge an Bestandteilen (a), (b) und (c) in der Oberflächenbeschichtungszusammensetzung vorliegen.

2. Beschichtungsflüssigkeit, die hergestellt wird durch Lösen oder Aufschließen der Oberflächenbeschichtungszusammensetzung gemäß Anspruch 1 in einem flüchtigen organischen Lösemittel.

3. Beschichtungsflüssigkeit gemäß Anspruch 2, wobei die Beschichtungsflüssigkeit eine nicht-wässrige Lösung ist.

4. Oberflächenbeschichtung, **dadurch gekennzeichnet, dass** sie anhand der Beschichtungsflüssigkeit gemäß Anspruch 2 oder 3 auf der Oberfläche eines Objekt-Basismaterials hergestellt wird.

5. Medizinisches Gerät, welches die Oberflächenbeschichtung gemäß Anspruch 4 aufweist.

6. Verfahren zur Bildung einer Oberflächenbeschichtung, welches das Auftragen und Trocknen der Beschichtungsflüssigkeit gemäß Anspruch 2 oder 3 auf der Oberfläche eines Objekt-Basismaterials umfasst.

7. Verfahren zur Bildung einer Oberflächenbeschichtung gemäß Anspruch 6, wobei eine Verbindung, die ein aktives Wasserstoffatom aufweist, nach praktischer Beendigung der Reaktion der Verbindungen (a), (b) und (c) der Oberflächenbeschichtungszusammensetzung zugegeben wird.

8. Verfahren zur Herstellung eines medizinischen Gerätes, welches das Auftragen und Trocknen der Beschichtungsflüssigkeit gemäß Anspruch 2 oder 3 auf der Oberfläche eines Objekt-Basismaterials umfasst.

9. Medizinisches Gerät, **dadurch gekennzeichnet, dass** es hergestellt wird durch das Verfahren zur Bildung einer Oberflächenbeschichtung gemäß Anspruch 6 oder das Verfahren zur Herstellung eines medizinischen Gerätes gemäß Anspruch 8.

**Revendications**

1. Composition de revêtement de surface, comprenant la composition suivante (A) :

(A) une composition contenant

(a) au moins un élément parmi un diisocyanate aromatique, un diisocyanate aliphatique et un diisocyanate alicyclique présent dans une quantité de 1 à 35 % en poids dans la composition de revêtement de surface,
(b) un polyol trifonctionnel ou fonctionnel plus élevé présent dans une quantité de 1 à 35 % en poids dans la composition de revêtement de surface, sélectionné dans un groupe composé de : polyols polymériques ramifiés de polyols à base de polyester ; poly(oxypropylène éther)polyol, poly(oxyéthylène-propylène éther)polyol et polytétraméthylène glycol ; polyols acryliques, huile de ricin, glycérol, triméthylolpropane, triméthyloléthane, 1,2,6,-héxanetriol, pentaérythritol, sorbitol et mannitol, et
(c) un polyéthylène glycol dans une quantité de 30 à 98 % en poids dans la composition de revêtement de surface, comprenant un poids moléculaire moyen de 4000 à 40000 ;

dans lequel les composés mentionnés ci-dessus (a), (b) et (c) sont présents par rapport à 100 % en poids de la quantité totale des composants (a), (b) et (c) dans la composition de revêtement de surface.

2. Liquide de revêtement préparé en faisant dissoudre ou suspendre la composition de revêtement de surface selon la revendication 1 dans un solvant organique volatile.

**3.** Liquide de revêtement selon la revendication 2, dans lequel le liquide de revêtement est une solution non aqueuse.

**4.** Revêtement de surface, **caractérisé en ce qu'**il est préparé en appliquant du liquide selon la revendication 2 ou la revendication 3 sur la surface d'un matériau de base d'objet.

**5.** Dispositif médical comprenant le revêtement de surface selon la revendication 4.

**6.** Procédé de former un revêtement de surface, comprenant les étapes d'appliquer et de faire sécher le liquide de revêtement selon la revendication 2 ou la revendication 3 sur la surface d'un matériau de base d'objet.

**7.** Procédé de former un revêtement de surface selon la revendication 6, dans lequel un composé ayant un atome d'hydrogène actif est ajouté à la composition de revêtement de surface après l'achèvement pratique de la réaction des composés (a), (b) et (c).

**8.** Procédé de fabrication d'un dispositif médical, comprenant les étapes d'appliquer et de faire sécher le liquide de revêtement selon la revendication 2 ou la revendication 3 sur la surface d'un matériau de base d'objet.

**9.** Dispositif médical, **caractérisé en ce qu'**il est produit à l'aide du procédé de former un revêtement de surface selon la revendication 6 ou à l'aide du procédé de fabrication d'un dispositif médical selon la revendication 8.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5076590 A
- JP 5168695 A
- JP 8033704 A
- JP 11022613 A
- JP 11506375 A
- JP 10231347 A
- JP 4227671 A

- US 5306798 A
- WO 9423771 A
- US 4876126 A
- US 6120904 A
- JP 2004280086 A
- WO 2006095766 A